# EUROPEAN PATENT SPECIFICATION

(11) **EP 1 867 732 B1**
(45) Date of publication and mention of the grant of the patent: **28.01.2015**
(21) Application number: 07018162.3
(22) Date of filing: 09.09.2002
(51) Int. Cl.: C12Q 1/00, C12Q 1/54, C12Q 1/32, A61B 5/00

(54) **Reagents, methods and devices for detecting analytes**
Reagenzien, Verfahren und Vorrichtungen zum Nachweis von Analyten
Réactifs, méthodes et dispositifs pour la détection des analytes

(30) Priority: 14.09.2001 US 318716 P
(43) Date of publication of application: 19.12.2007
(62) Divisional of application: 02019843.8
(73) Proprietor: Bayer HealthCare LLC, Whippany, NJ 07981-0915 (US)
(72) Inventor: Vreeke, Mark S., Houston TX 77057 (US); Warchal-Windham, Mary Ellen, Osceola IN 46561 (US); Blaschke, Christina, White Pigeon MI 49099 (US); Mikel, Barbara J., Mishawaka IN 46544 (US); Cooper, Howard A., Elkhart IN 46516 (US)
(74) Representative: BIP Patents

(56) References cited:
- EP-A- 0 330 517
- EP-A- 1 130 111
- WO-A-92/07263
- WO-A-92/07953
- US-A- 4 711 245
- US-A- 5 520 786

## Description

### BACKGROUND

The present invention relates to electrochemical sensors for measurement of analytes and, more particularly, to electrochemical sensors for the measurement of glucose in the blood.

The monitoring of certain analyte concentrations in the body enables early detection of health risks, and identifies the need for the introduction of therapeutic measures. One of the most commonly monitored analytes is glucose, the blood concentration of which is important in determining the appropriate dosages of insulin for diabetics. Various methods have been developed for monitoring glucose levels in the blood, including the use of electrochemical biosensors. Electrochemical biosensors are based on enzyme-catalyzed chemical reactions involving the analyte of interest. In the case of glucose monitoring, the relevant chemical reaction is the oxidation of glucose to gluconolactone. This oxidation is catalyzed by a variety of enzymes, some of which may contain a bound coenzyme such, as nicotinamide adenine dinucleotide (phosphate) (NAD(P)), while others may contain a bound cofactor such as flavin adenine dinucleotide (FAD) or pyrroloquinolinequinone (PQQ).

In biosensor applications, the redox equivalents generated in the course of the oxidation of glucose are transported to the surface of an electrode whereby an electrical signal is generated. The magnitude of the electrical signal is then correlated with concentration of glucose. The transfer of redox equivalents from the site of chemical reaction in the enzyme to the surface of the electrode is accomplished with the use of electron transfer mediators.

A significant problem with the use of electron transfer mediators in biosensors is the instability of these compounds upon exposure to common environmental conditions such as temperature and moisture. As a result, the number of mediators suitable for use in glucose biosensors is quite limited.

U.S. Pat. No. 5,520,786 ('786) to Bloczynski et al. describes families of phenothiazine and phenoxazine compounds suitable for use as electron transfer mediators with the enzymes dihydronicotinamide adenine dinucleotide (NADH), NADPH, and analogs thereof. Cofactor based enzymes such as FAD-glucose oxidase and PQQ-glucose dehydrogenase have several advantages over NAD-based enzymes, including lower cost, higher enzyme activity, increased stability, and bound as opposed to readily dissociable cofactor.

Electron transfer mediators previously used with FAD-glucose oxidase and PQQ-glucose dehydrogenase include quinones, phenzine methosulfate, dichlorophenolindophenol and ferricyanide. Unfortunately, these compounds have proven to be highly susceptible to the environmental agents described above, and result in biosensor reagents of low stability. Thus, mediators are needed which exhibit good stability upon exposure to commonly-encountered environmental agents, and which can be used in flavoprotein- and quinoprotein-based systems.

In addition to the need for biosensor reagents that are stable to the environmental agents described above, it would be desirable to provide biosensor reagents that are stable to the radiation conditions commonly employed in lancet sterilization. Reagents stable to such radiation sterilization could be incorporated into highly user-convenient units in which lancet and biosensor are combined.

The present invention is directed to electron transfer mediators for use in flavoprotein- and quinoprotein-based biosensor reagents, which exhibit improved stability to both environmental interferents and to radiation sterilization.

### SUMMARY

The scope of the present invention is defined solely by the appended claims, and is not affected to any degree by the statements within this summary. By way of introduction, the presently preferred embodiments described herein are directed towards remedying the aforementioned stability problems of electron transfer mediators and enzyme biosensors.

Briefly stated, a composition aspect of the present invention is directed to a reagent for detecting an analyte, comprising (a) an enzyme selected from the group consisting of a flavoprotein, a quinoprotein, and a combination thereof; and (b) a mediator selected from the group consisting of 3-(3',5'-dicarboxy-phenylimino)-3H-phenoxazine and 3-(3',5'-phenylimino)-3H-phenothiazinedisulfonic acid, and a combination thereof.

A first apparatus aspect of the present invention is directed to an electrochemical sensor comprising: (a) a working electrode having a surface; and (b) a second electrode coupled to the working electrode. The surface of the working electrode is coated with a solution or mixture of a reagent comprising an enzyme selected from the group consisting of a flavoprotein, a quinoprotein, and a combination thereof; and a mediator selected from the group consisting of 3-(3',5'-dicarboxy-phenylimino)-3H-phenoxazine, 3-(3',5'-phenylimino)-3H-phenothiazinedisulfonic acid, and a combination thereof.

A second apparatus aspect of the present invention is directed to a device for measuring an analyte, comprising (a) a lancet; and (b) a sampling chamber connected to the lancet. The sampling chamber comprises a reagent comprising an enzyme selected from the group consisting of PQQ-glucose dehydrogenase, FAD-glucose oxidase, and a combination thereof; and (b) a mediator selected from the group consisting of 3-(3',5'-dicarboxy-phenylimino)-3H-phenoxazine, 3-(3',5'-phenylimino)-3H-phenothiazinedisulfonic acid, and a combination thereof.

Disclosed herein is a first method of producing a sterilized device for measuring an analyte, comprising (a) providing a device in accordance with the present invention, and (b) irradiating the device with E-beam or gamma ray radiation.

Also disclosed herein is a second method aspect of the present invention is directed to a method for detecting an analyte which undergoes a chemical reaction, the method comprising (a) providing an electrode surface; (b) catalyzing the chemical reaction with an enzyme selected from the group consisting of a flavoprotein, a quinoprotein, and a combination thereof; (c) generating a redox equivalent by the chemical reaction; and (d) transferring the redox equivalent to the electrode surface using a mediator selected from the group consisting of 3-(3',5'-dicarboxy-phenylimino)-3H-phenoxazine, 3-(3',5'-phenylimino)-3H-phenothiazinedisulfonic acid, and a combination thereof.

The presently preferred embodiments discussed herein may possess one or more advantages relative to other flavoprotein- and quinoprotein-based biosensor reagents, which can include but are but not limited to: improved biosensor reagent stability; enhanced electron transfer capability of mediators; ability to tune mediators for optimum electrode operation; reduced oxygen susceptibility of mediators; increased thermal stability of mediators; increased stability of mediators to ambient humidity; lower redox potential of mediators; reduced susceptibility to interferents in blood; and stability of biosensor reagents to radiation sterilization conditions.

### BRIEF DESCRIPTION OF THE DRAWINGS

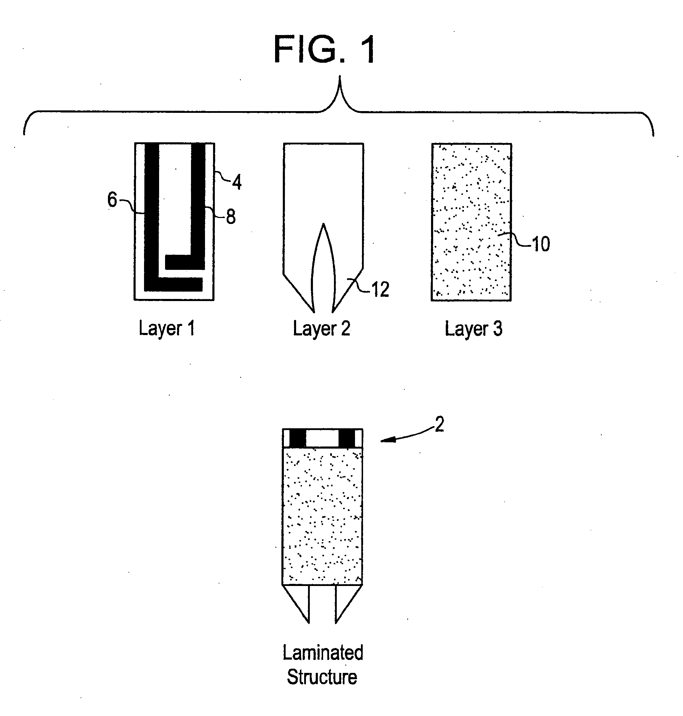

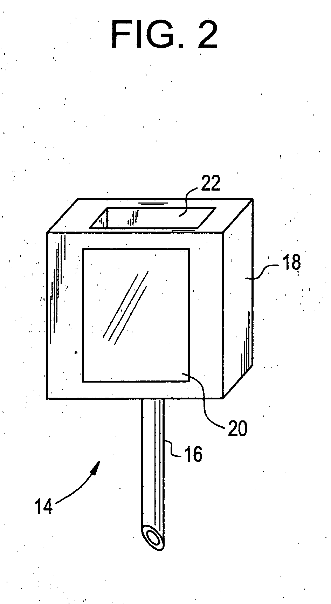

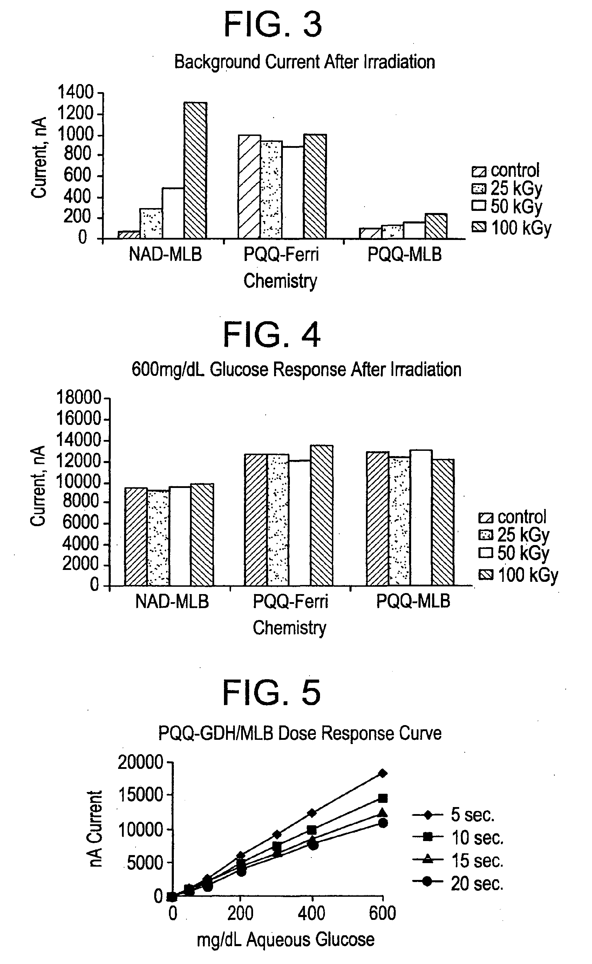

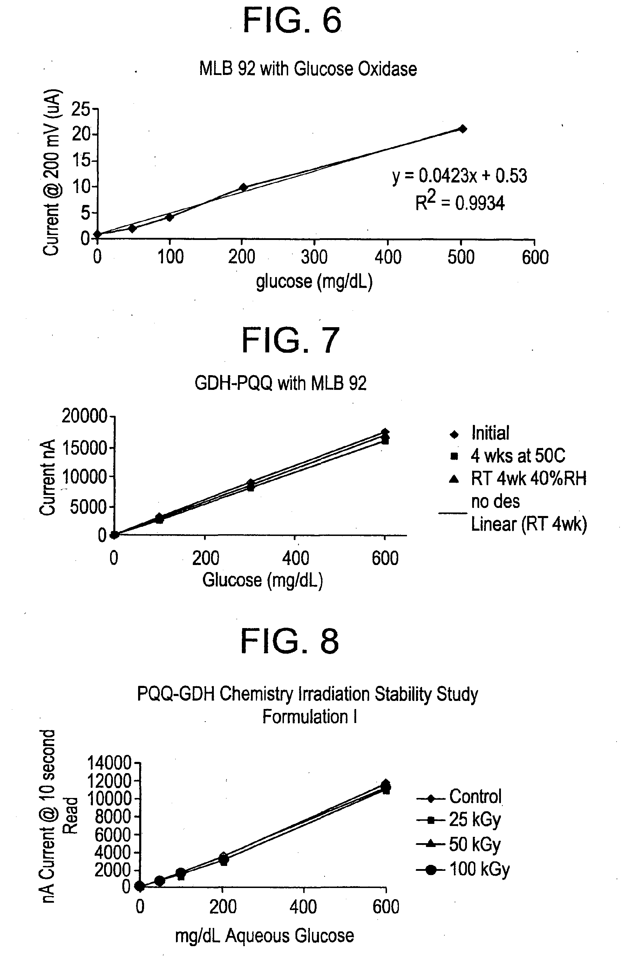

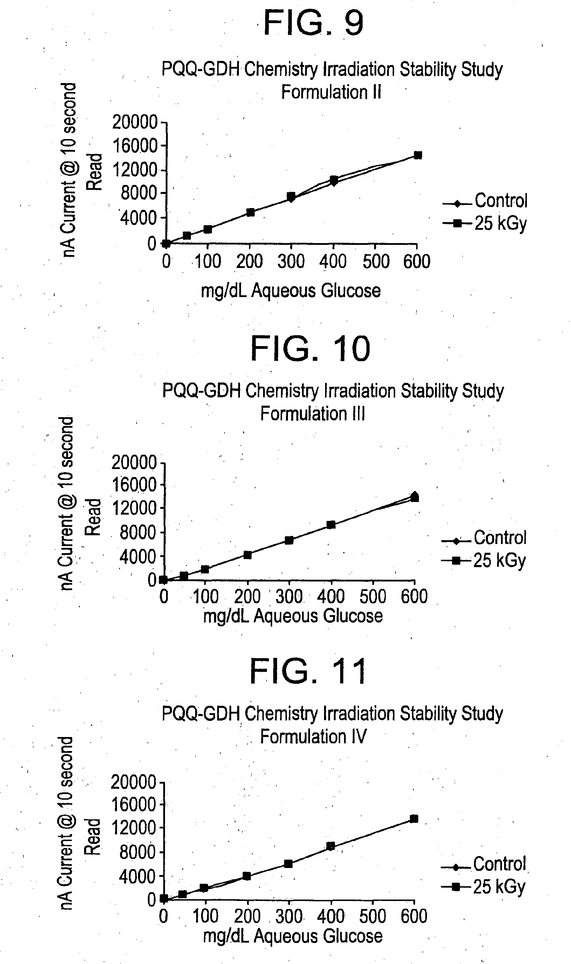

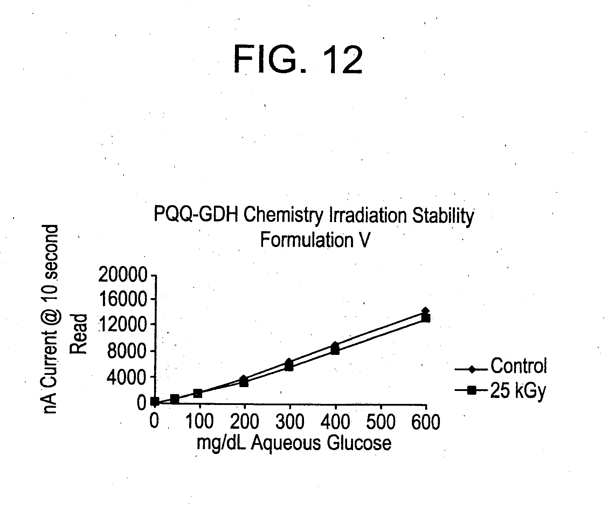

FIG. 1 shows a schematic illustration of a device for measuring an analyte that embodies features of the present invention. FIG. 2 shows a perspective view of an integrated lancet/biosensor device for use in accordance with the present invention. FIG. 3 shows a graph of background currents for 3 formulations of biosensor reagents exposed to increasing levels of radiation. FIG. 4 shows a graph of the current response of radiation sterilized biosensor reagents upon exposure to glucose. FIG; 5 shows a plot of current vs. glucose concentration at increasing time intervals for a PQQ-glucose dehydrogenase/ phenothiazine biosensor. FIG. 6 shows a plot of current vs. glucose concentration for a [FAD]-glucose oxidase/phenothiazine biosensor. FIG. 7 shows a plot of current vs. glucose concentration for a PQQ-glucose dehydrogenase/phenothiazine biosensor reagent subjected to heat stress and humidity stress. FIGS. 8-12 show plots of current vs. glucose concentration for 5 formulations of PQQ-glucose dehydrogenase/phenothiazine biosensors exposed to varying levels of radiation.

### DETAILED DESCRIPTION OF THE PRESENTLY PREFERRED EMBODIMENTS

Throughout this description and in the appended claims, the following definitions are to be understood: The term "analyte" refers to one or a plurality of species having a concentration of interest. The term "flavoprotein" refers to enzymes containing flavin cofactors. The term "quinoprotein" refers to enzymes containing PQQ or similar cofactors. The phrase "redox equivalent" refers to one or a plurality of charged species (e.g., electrons) produced in electrochemical reactions involving the analyte. The phrase "E-beam irradiation" or "electron beam irradiation" refers to a process of exposure to a concentrated, high-current stream of electrons. The terms "alkyl," "alkenyl," "alkynyl," "aryl," "heteroaryl," "cyclic," "heterocyclic," "halo," "haloalkyl," "carboxy," "carboxyalkyl," "alkoxycarbonyl," "aryloxycarbonyl," "aromatic keto," "aliphatic keto," "alkoxy," "aryloxy," "nitro," "dialkylamino," "aminoalkyl," "sulfo," "dihydroxyboron," and the like refer to substituents well known in the art, which may be branched or unbranched and may themselves be substituted with one or more substituents. The phrase "biosensor reagent" refers to the combination of an enzyme that catalyzes a reaction of an analyte, and a phenothiazine and/or phenoxazine mediator. The term "bioburden" refers to the population of viable microorganisms on a product determined immediately prior to irradiation.

A biosensor reagent for detecting an analyte in accord with the present invention includes (1) an enzyme selected from the group consisting of a flavoprotein, a quinoprotein, and a combination thereof; and (2) a mediator selected from the group consisting of 3-(3',5'-dicarboxy-phenylimino)-3H-phenoxazine, 3-(3',5'-phenylimino)-3H-phenothiazinedisulfonic acid, and a combination thereof.

The nature of the analyte monitored in accord with the present invention is unrestricted, provided the analyte undergoes a chemical reaction that is catalyzed by an enzyme selected from the group consisting of a flavoprotein, a quinoprotein, and a combination thereof. Preferred analytes include but are not limited to glucose, lactate, D-amino acids, ascorbate, alcohol, cholesterol, choline, and acetylcholine.

Flavoproteins in accord with the present invention include FAD-glucose oxidase (Enzyme Classification No. 1.1.3.4), Flavin-hexose oxidase (EC No. 1.1.3.5) and FAD-glucose dehydrogenase (EC No. 1.1.99.10) For information relating to these flavoproteins, see: Adriaan Joseph Jan Olsthoorn, "Structural and Mechanistic Aspects of Soluble Quinoprotein Glucose Dehydrogenase from Acinetobacter calcoaceticus," Ph.D. dissertation, Delft University of Technology, The Netherlands, 1999. Additional oxidase enzymes for use in accord with the present invention include but are not limited to lactate oxidase, cholesterol oxidase, alcohol oxidase (e.g., methanol oxidase), d-aminoacid oxidase, choline oxidase, and FAD derivatives thereof. A preferred flavoprotein for use in accord with the present invention is FAD-glucose oxidase.

Quinoproteins in accord with the present invention include but are not limited to membrane bound and soluble PQQ-glucose dehydrogenase (EC No. 1.1.99.17). Information relating to PQQ-glucose dehydrogenase can be found in the Olsthoorn reference cited above. Additional quinoprotein enzymes for use in accord with the present invention include but are not limited to lactate dehydrogenase, aldehyde dehydrogenase, methylamine dehydrogenase, alcohol dehydrogenase (e.g., methanol dehydrogenase), and PQQ derivatives thereof. A preferred quinoprotein for use in accord with the present invention is PQQ-glucose dehydrogenase.

Mediators in accord with the present invention include phenothiazines having the formula 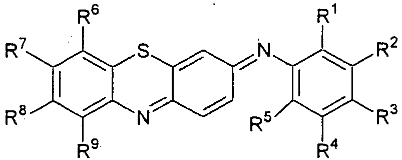 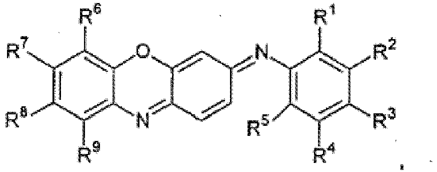 wherein R3, R5, R6, R7, R8, and R9 are hydrogen, and (i) either R2 and R3 are both hydrogen and R1 and R4 are both a sulfonic acid or (ii) R1 and R5 are both hydrogen and R2 and R4 are both a carboxylic acid.

In contrast to the single electron transfer carrying capability of K3Fe(CN)6, mediators in accord with the present invention have the ability to carry two redox equivalents, and are therefore well suited for use in FAD and quinoprotein oxidation/reduction processes, which generally involve the transfer of two electrons. Moreover, the potential of mediators of the present invention can be tuned to the optimum potential (i.e., the potential where the signal contribution from interferences is minimized) for a specific sample matrix by varying the substitution on the aromatic rings. Electron-donating substituents (e.g., alkyl, alkoxy, amine, hydroxy, etc.) result in decreased redox potentials, while electron-withdrawing substituents (e.g., carboxylic acid, ester, aldehyde, ketone, nitrile, nitro, sulfonic acid, trifluromethyl, etc.) result iri increased redox potentials. For blood or plasma samples, the ideal potential usually lies between about -200 and about 100 mV versus an Ag/AgCl reference.

The substituents on the aromatic rings, in addition to their utility in tuning the redox potentials of the mediators, can also be used to enhance mediator solubility. For example, the introduction of a substituent having the capacity for hydrogen bonding can be expected to render the mediator more water soluble than a mediator lacking such substitution. In addition, these substituents can serve as functional groups for immobilizing the mediators to a support (e.g., the electrode surface or, alternatively, a chemical matrix such as a polymer backbone, which is suitable for application to the electrode surface).

The mediators used in biosensor reagents according to the present invention are 3-(3',5'-dicarboxy-phenylimino)-3H-phenoxazine, 3-(3',5'-phenylimino)-3H-phenothiazinedisulfonic acid.

More preferably, the mediator used in accord with the present invention

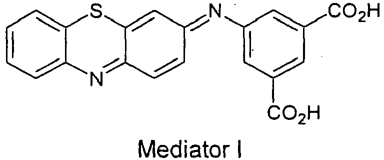

Relative to ferricyanide, phenothiazine mediators-in particular mediator I-are less susceptible to oxygen degradation, more thermally stable, and more stable to ambient humidity. In addition, mediator I works at a lower redox potential than ferricyanide. For example, Eo for mediator I is approximately 0 mV versus an Ag/AgCl reference, whereas Eo for ferricyanide is approximately 250 mV versus an. Ag/AgCl reference. The lower redox potential of phenothiazine mediators is advantageous in that there is a region around 0 mV versus an Ag/Agcl reference in which the amount of electrochemical interferences are minimized. Thus, the impact from chemical interferents in the blood can be minimized by using these mediators.

Reagents embodying features of the present invention can be incorporated into a variety of biosensor devices, including but not limited to the ones described in United States Patents No. 5,120,420 and No. 5,798,031, the entire contents of both of which are incorporated herein by reference, except that in the event of any inconsistent disclosure or definition from the present application, the disclosure or definition herein shall be deemed to prevail.

Turning now to the drawings, FIG. 1 shows a representative electrochemical sensor in accordance with the present invention. The electrochemical sensor 2 is comprised of an insulating base 4 upon which is printed (typically by screen printing techniques) an electrode pattern (6 and 8) and a reagent layer 10 that contains a reagent embodying features of the present invention. The two parts of the electrode print, 6 and 8, provide the working and reference electrodes necessary for the electrochemical determination. A lancet element 12 can be incorporated into the electrochemical sensor (e.g., interposed between layers 1 and 2), as is described more fully hereinbelow. The three layers shown in FIG. 1 can be joined by means of an adhesive (e.g., pressure sensitive, hot melt, etc.) or by sonic welding, depending on the identity of the materials.

It has been found that biosensor reagents comprising PQQ-glucose dehydrogenase and certain phenothiazine mediators exhibit high stability to radiation sterilization. A preferred application of radiation stable biosensor reagents in accord with the present invention is for the development of integrated lancet/biosensor devices. An example of such an integrated device is illustrated in FIG. 2 and described in United States Patent No. 5,801,057, the entire contents of which are incorporated herein by reference, except that in the event of any inconsistent disclosure or definition from the present application, the disclosure or definition herein shall be deemed to prevail.

As shown in FIG. 2, the integrated lancet/biosensor device **14** has a finely bored needle **16** connected to a sampling chamber **18**. Sampling chamber **18** has at least one optical window **20** and a vent **22** through which air can be displaced when the chamber **18** fills with blood or other fluids. Preferably, sampling chamber **18** comprises a biosensor reagent comprising PQQ-glucose dehydrogenase and a phenothiazine and/or phenoxazine mediator. Preferably, the mediator has a structure represented by mediator I above. Once sampling chamber **18** has been loaded with biosensor reagent, the entire device **14** can be subjected to radiation sterilization. Preferably, the method of sterilization involves electron beam (E-beam) irradiation or gamma irradiation.

As set forth in the Association for the Advancement of Medical Instrumentation document ANSI/AAMI/ISO 11137 -1994, products that penetrate the skin and come into contact with the blood must have a sterility assurance level (SAL) of 10⁻⁶, which corresponds to a one in a million probability of a viable microorganism being present on a product unit after sterilization. The sterilization dose needed to achieve a 10⁻⁶ SAL depends on the bioburden of the sample. For example, a sample with a bioburden of 1,021 requires a sterilization dose of 24.9 kGy to achieve a 10⁻⁶ SAL.

In the examples described hereinbelow, electron beam (E-beam) irradiation was employed as the method of sterilization. The biosensor reagents subjected to the electron beam absorb energy from the electrons. The energy that is absorbed per unit mass of material is referred to as the absorbed dose, and it is this absorption of energy-or dose delivery-that destroys the reproductive cells and DNA chains of microorganisms, thereby rendering a product sterile. E-beam doses of 25, 50 and 100 kGy were used because the bioburden of the biosensor reagents was unknown.

FIG. 3 shows a graph of the background currents observed for three formulations of biosensor reagents exposed to increasing levels of radiation: (1) NAD-glucose dehydrogenase with Mediator I, (2) PQQ-glucose dehydrogenase with Ferricyanide, and (3) PQQ-glucose dehydrogenase with Mediator I. The PQQ formulations tolerated the irradiation extremely well. In contrast, the NAD formulation exhibited poor tolerance to the sterilization conditions, and resulted in a background signal which constituted a significant amount of the glucose signal. While formulation (2) exhibited good tolerance to the radiation process, the activity of the extracted enzyme was lower than the corresponding activity of the enzyme extracted from formulation (3). Fig 4 shows a graph of current response when these radiation-sterilized sensors were exposed to 600 mg/dL glucose.

The manner in which a device embodying features of the present invention is made, and the process by which such a device is used for monitoring an analyte, will be abundantly clear to one of ordinary skill in the art based upon joint consideration of both the preceding description, and the following representative procedures. It is to be understood that many variations in the presently preferred embodiments illustrated herein will be obvious to one of ordinary skill in the art, and remain within the scope of the appended claims.

For example, the working electrode employed in electrochemical sensors according to the present invention can be varied, with suitable electrodes including but not limited to carbon electrodes, platinum electrodes, palladium electrodes, gold electrodes, and the like. Similarly, the reference electrode can be varied, with suitable electrodes including but not limited to silver-silver chloride electrodes, calomel electrodes, saturated calomel electrodes, and the like. Alternatively, a quasi-reference electrode (e.g., a large surface area platinum electrode) of the type commonly used in nonaqueous electrochemical experiments (i.e., an electrode which does not have a specific redox species to which its potential is referenced) can be used in accord with the present invention. The surface areas of all electrodes employed in accordance with the present invention are likewise subject to variation. Preferably, the working electrode has dimensions of about 0.6 mm x 1.2 mm.

Furthermore, the compositions and pH of the buffer solutions employed, and the enzyme activities and concentrations of components of the biosensor reagents, are subject to wide variation. Suitable buffer solutions include but are not limited to HEPES (i.e., N-2-hydroxyethylpiperazine-N'-2-ethanesulfonic acid), MOPS (i.e., 3-(N-morpholino)propanesulfonic acid), TES (i.e., N-tris(hydroxymethyl)methyl-2-aminoethanesulfonic acid), 2-([2-hydrox-1,1-bis(hydroxymethyl)-ethyl]amino)ethanesulfonic acid), PIPES (i.e., piperazine-N,N'-bis(2-ethanesulfonic acid)), 1,4-piperazinediethanesulfonic acid), ACES (i.e., N-(carbamoylmethyl)-2-aminoethanesulfonic acid), N-(2-acetamidol)-2-aminoethanesulfonic acid, BES (i.e., N,N-bis(2-hydroxyethyl)-2-aminoethanesulfonic acid, and Dulbecco's buffer (i.e., 0.008M sodium phosphate, 0.002M potassium phosphate, 0.14M sodium chloride, 0.01 M potassium chloride, pH 7.4).

The manner in which reagents and devices embodying features of the present invention are made, and the methods by which these reagents and devices are used for monitoring an analyte, will be abundantly clear to one of ordinary skill in the art based upon joint consideration of both the preceding description, and the following representative procedures.

While the examples provided hereinbelow relate to in vitro applications of the biosensor reagents in accord with the present invention, it is contemplated that these reagents can also be adapted for in vivo analyte monitoring by chemically immobilizing the phenoxazine and/or phenothiazine mediators (e.g., by chemical reaction at one or more of the substituent groups on the aromatic rings), and incorporating the immobilized mediators into a device which can be implanted subcutaneously into a patient.

### EXAMPLES

### Preparation of Biosensor and Glucose Dose-Response

A liquid chemistry reagent was prepared to be 20 Units/µL pyrolloquinolinequinone-glucose dehydrogenase (PQQ-GDH) and 24 mM mediator I in 100 mM Sodium Phosphate, pH 7.4. The first component of the reagent was made by dissolving the mediator in 100 mM phosphate pH 7.4, adjusting the pH back to 7.4, and filtering the solution by forcing it through a Whatman 0.45 micron PTFE syringe filter. The reagent was completed by adding lyophilized PQQ-GDH (Toyobo Product No. GLD-321) to an activity of 20 U/µL.

The chemistry formulation was deposited onto electrodes, which had been produced using a 3-pass screen-printing process by Conductive Technologies, Inc. During this process, the silver/silver chloride (DuPont 5870 ink) leads and reference electrode were printed first onto polycarbonate base material. The second pass of Dupont 7102T carbon-graphite working electrode was printed on top of this. A final pass of Norcote RDMSK4954-A2 dielectric defined the working electrode area to be 0.0113 cm².

The chemistry was deposited over the working electrode with the use of an Asymtek Automove^{®} 402 Dispensing System. The system was programmed to perform the transfer by dipping a 62 mL stainless steel pin into a 1.5 mL Eppendorf vial filled with reagent. Polycarbonate lid material was laminated to the sensors creating a capillary area over the working and reference electrodes capable of holding approximately 3 µL of test solution. The capillary area, which defines the sample volume, is first formed in the polycarbonate lid material by a coining or stamping process.

As shown in FIG. 5, reactivity of the chemistry was analyzed by generating a glucose dose-response curve with buffered (100 mM phosphate, 100 mM sodium chloride, pH 7.4) samples containing a range of glucose concentrations from 0 to 600 mg/dL. Current generated at each of the glucose concentrations was measured using a potentiostat programmed to apply 150 mV potential with trigger level set to 100 nA, and timing programmed to record the current at 5,10,15, and 20 seconds. The trigger level refers to a threshold level above which timing and recording are initiated.

Sensors formulated with 20U Glucose Oxidase/sensor and 6 mM mediator I were deposited onto electrode sensors as above. The dose response plot shown in FIG. 6 was obtained.

### Preparation of Electrochemical Biosensor and Heat/Humidity Stability

Electrochemical biosensors were constructed using a screen-printing process. Sensors were comprised of a carbon working electrode and a silver/silver chloride reference electrode. A solution (150 to 800nl) containing 12mM mediator I in 100mM phosphate buffer (pH 7.4), and of the enzyme PQQ-glucose dehydrogenase (10 U/µL) was deposited on the surface of the working electrode and allowed to dry at room temperature for 5 minutes prior to desiccation. The electrodes were assembled into a format having a small capillary gap, which allowed inoculation of the sensors with sample solutions.

In subsequent tests, the sensors were subjected to the following environmental conditions prior to testing: 1) 50 °C for 2, 4, and 8 weeks; and 2) room temperature with 40% relative humidity. The sensors were poised at a potential of 150mV relative to the Ag/AgCl reference electrode and the resulting current was measured. This mediator/enzyme combination is quite stable to both heat stress and humidity stress as shown in FIG. 7.

### Sterilization of Biosensors and Radiation Stability Data

Five formulations of biosensor reagents (Table 1) were prepared and subjected to E-beam irradiation using SureBeam^{®} sterilization technology at Titan Scan Technologies (San Diego, CA). Formulation I was irradiated at 25 kGy, 50 kGy, and 100 kGy, whereas each of Formulations II-V was irradiated at 25 kGy only. In the two rightmost column headings of Table 1, the abbreviation CMC refers to carboxymethylcellulose, and the abbreviation PEO refers to polyethylene oxide.

**Table 1**

| Formulation # | Enzyme Concentration PQQ-GDH Units | Concentration Mediator I mM | Polymer Concentration CMC % | Polymer Concentration PEO % |
|---|---|---|---|---|
| I | 20 | 12 | 0 | 0 |
| II | 20 | 12 | 0 | 0 |
| III | 20 | 12 | 1 | 0 |
| IV | 20 | 12 | 2 | 0 |
| V | 20 | 12 | 0 | 2 |

FIGS. 8-12 show glucose dose response curves for each of the five formulations both before and after irradiation. The stability of the five formulations is high, as is clearly shown by the near overlapping of the glucose response generated before and after irradiation.

Table 2 shows the results of enzyme assays conducted on the five formulations both before and after irradiation. Enzyme activity following irradiation remains high in all instances.

**Table 2**

| Formulation # | kGy Level | Enzyme Activity |
|---|---|---|
| I | 0 | 4.67 |
| | 25 | 4.32 |
| | 50 | 4.20 |
| | 100 | 4.24 |
| II | 0 | 3.31 |
| | 25 | 3.34 |
| III | 0 | 4.93 |
| | 25 | 4.87 |
| IV | 0 | 4.96 |
| | 25 | 4.86 |
| V | 0 | 3.63 |
| | 25 | 4.05 |

The foregoing detailed description and examples have been provided by way of explanation and illustration, and are not intended to limit the scope of the appended claims. Many variations in the presently preferred embodiments illustrated herein will be obvious to one of ordinary skill in the art, and remain within the scope of the appended claims.

## Claims

1. An electrochemical sensor, comprising:
a first electrode having a surface; and
a second electrode coupled to the first electrode,
wherein the surface of the first electrode includes a reagent comprising
an enzyme selected from the group consisting of a flavoprotein, a quinoprotein, or a combination thereof; and
a mediator selected from the group consisting of 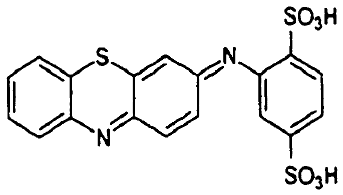 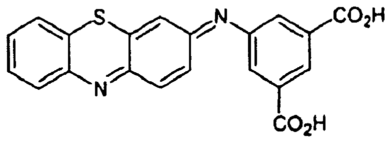 or a combination thereof.

2. The electrochemical sensor of claim 1, further comprising a polymer selected from the group consisting of carboxymethylcellulose, polyethylene oxide, and combinations thereof.

3. The electrochemical sensor of claim 1, wherein the buffer is selected from the group consisting of sodium phosphate, potassium phosphate, Hepes, MOPS, TES, Pipes, ACES, BES, Dulbecco's, and combinations thereof.

4. The electrochemical sensor of claim 1, wherein the reagent is stable after exposure to irradiation.

5. The electrochemical sensor of claim 4, wherein the irradiation is one of electron-beam radiation, gamma radiation, and ultraviolet radiation.

6. The electrochemical sensor of claim 1, wherein the enzyme is selected from the group consisting of FAD-glucose dehydrogenase, PQQ-glucose dehydrogenase, and a combination thereof.

7. An electrochemical sensor for detecting an analyte, comprising:
a flavoprotein enzyme; and
a mediator comprising

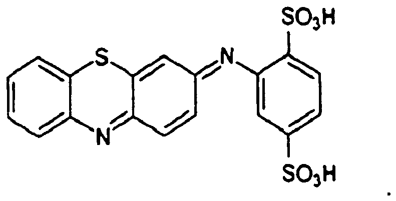

8. The electrochemical sensor of claim 7, further comprising a polymer selected from the group consisting of carboxymethylcellulose, polyethylene oxide, and combinations thereof.

9. The electrochemical sensor of claim 7, wherein the flavoprotein is selected from the group consisting of FAD-glucose oxidase, flavin-hexose oxidase, FAD-glucose dehydrogenase, FAD-lactate oxidase, FAD-cholesterol oxidase, FAD-alcohol oxidase, FAD-d-aminoacid oxidase, FAD-choline oxidase, and combinations thereof.

10. The electrochemical sensor of claim 7, wherein the reagent is stable after exposure to irradiation.

## Patentansprüche

1. Elektrochemischer Sensor, der aufweist:
eine erste Elektrode mit einer Oberfläche; und
eine zweite Elektrode, die mit der ersten Elektrode gekoppelt ist,
wobei die Oberfläche der ersten Elektrode ein Reagenz enthält, das ein Enzym umfasst, das aus der Gruppe ausgewählt ist, die aus einem Flavoprotein, einem Quinoprotein oder aus einer Kombination davon besteht; und
einen Mediator, der aus der Gruppe ausgewählt ist, die aus 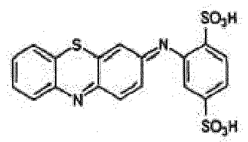 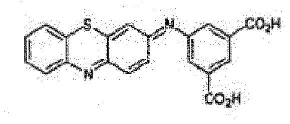 oder aus einer Kombination davon besteht.

2. Elektrochemischer Sensor nach Anspruch 1, der ferner ein Polymer aufweist, das aus der Gruppe ausgewählt ist, die aus Carboxymethylcellulose, Polyethylenoxid und Kombinationen davon besteht.

3. Elektrochemischer Sensor nach Anspruch 1, wobei der Puffer aus der Gruppe ausgewählt ist, die aus Natriumphosphat, Kaliumphosphat, Hepes, MOPS, TES, Pipes, ACES, BES, Dulbecco's und Kombinationen davon besteht.

4. Elektrochemischer Sensor nach Anspruch 1, wobei das Reagenz nach einer Aussetzung gegenüber einer Bestrahlung stabil ist.

5. Elektrochemischer Sensor nach Anspruch 4, wobei die Bestrahlung eine aus einer Elektronenstrahlstrahlung, Gammastrahlung und aus einer ultravioletten Strahlung ist.

6. Elektrochemischer Sensor nach Anspruch 1, wobei das Enzym aus der Gruppe ausgewählt ist, die aus FAD-Glucose-Dehydrogenase, PQQ-Glucose-Dehydrogenase und aus einer Kombination davon besteht.

7. Elektrochemischer Sensor zum Detektieren eines Analyten, der aufweist:
ein Flavoproteinenzym; und
einen Mediator, der umfasst:

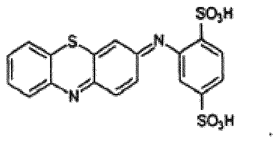

8. Elektrochemischer Sensor nach Anspruch 7, der ferner ein Polymer aufweist, das aus der Gruppe ausgewählt ist, die aus Carboxymethylcellulose, Polyethylenoxid und aus Kombinationen davon besteht.

9. Elektrochemischer Sensor nach Anspruch 7, wobei das Flavoprotein aus der Gruppe ausgewählt ist, die aus FAD-Glucoseoxidase, Flavin-Hexoseoxidase, FAD-Glucose-Dehydrogenase, FAD-Lactatoxidase, FAD-Cholesterinoxidase, FAD-Alkoholoxidase, FAD-d-Aminosäureoxidase, FAD-Cholinoxidase und aus Kombinationen davon besteht.

10. Elektrochemischer Sensor nach Anspruch 7, wobei das Reagenz nach einer Aussetzung gegenüber einer Bestrahlung stabil ist.

## Revendications

1. Capteur électrochimique, comprenant :
une première électrode ayant une surface ; et
une deuxième électrode couplée à la première électrode,
la surface de la première électrode comportant un réactif comprenant une enzyme choisie dans le groupe constitué par une flavoprotéine, une quinoprotéine, et les combinaisons de celles-ci ; et
un médiateur choisi dans le groupe constitué par 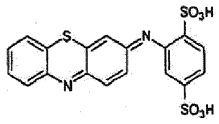 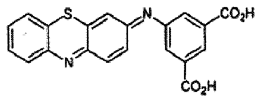 et les combinaisons de ceux-ci.

2. Capteur électrochimique selon la revendication 1, comprenant en outre un polymère choisi dans le groupe constitué par la carboxyméthylcellulose, l'oxyde de polyéthylène, et les combinaisons de ceux-ci.

3. Capteur électrochimique selon la revendication 1, dans lequel le tampon est choisi dans le groupe constitué par le phosphate de sodium, le phosphate de potassium, l'Hepes, le MOPS, le TES, le Pipes, l'ACES, le BES, le milieu de Dulbecco, et les combinaisons de ceux-ci.

4. Capteur électrochimique selon la revendication 1, dans lequel le réactif est stable après exposition à une irradiation.

5. Capteur électrochimique selon la revendication 4, dans lequel l'irradiation est un rayonnement de faisceau d'électrons, ou un rayonnement gamma, ou un rayonnement ultraviolet.

6. Capteur électrochimique selon la revendication 1, dans lequel l'enzyme est choisie dans le groupe constitué par la FAD-glucose déshydrogénase, la PQQ-glucose déshydrogénase, et les combinaisons de celles-ci.

7. Capteur électrochimique pour la détection d'un analyte, comprenant :
une enzyme flavoprotéine ; et
un médiateur comprenant

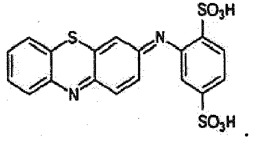

8. Capteur électrochimique selon la revendication 7, comprenant en outre un polymère choisi dans le groupe constitué par la carboxyméthylcellulose, l'oxyde de polyéthylène, et les combinaisons de ceux-ci.

9. Capteur électrochimique selon la revendication 7, dans lequel la flavoprotéine est choisie dans le groupe constitué par la FAD-glucose oxydase, la flavine-hexose oxydase, la FAD-glucose déshydrogénase, la FAD-lactate oxydase, la FAD-cholestérol oxydase, la FAD-alcool oxydase, la FAD-d-aminoacide oxydase, la FAD-choline oxydase, et les combinaisons de celles-ci.

10. Capteur électrochimique selon la revendication 7, dans lequel le réactif est stable après exposition à une irradiation.
